# EUROPÄISCHE PATENTSCHRIFT

(11) **EP 1 200 070 B1**
(45) Veröffentlichungstag und Bekanntmachung des Hinweises auf die Patenterteilung: **06.08.2003**
(21) Anmeldenummer: 00949281.0
(22) Anmeldetag: 07.07.2000
(51) Int. Cl.: A61K 9/20, A61K 31/135

(54) **AMBROXOLHALTIGE LUTSCHTABLETTE**
AMBROXOL-CONTAINING LOZENGE
PASTILLE A SUCER CONTENANT DE L'AMBROXOL

(30) Priorität: 20.07.1999 DE 19933148
(43) Veröffentlichungstag der Anmeldung: 02.05.2002
(60) Teilanmeldung: 03013014.0
(73) Patentinhaber: Boehringer Ingelheim International GmbH, 55216 Ingelheim am Rhein (DE)
(72) Erfinder: MÄRZ, Frieder, D-55270 Soergenloch (DE); VON DER HEYDT, Holger, Hans-Hermann, D-55232 Alzey (DE); SCHMITT, Horst, D-55437 Nieder-Hilbersheim (DE)
(86) Internationale Anmeldenummer: EP0006437
(87) Internationale Veröffentlichungsnummer: WO01005378

(56) Entgegenhaltungen:
- EP-A- 0 138 020
- EP-A- 0 546 846
- EP-A- 0 687 464
- DE-A- 3 034 975
- PHARMAZEUTISCHE INDUSTRIE, Bd. 50, Juli 1988 (1988-07), Seiten 839-845, XP000971106 in der Anmeldung erwähnt

## Beschreibung

Die vorliegende Erfindung betrifft eine neuartige, den Wirkstoff Ambroxol enthaltende Lutschtablette mit verbesserten Eigenschaften.

Das unter dem internationalen Freinamen Ambroxol bekannte trans-4-(2-Amino-3,5-dibrombenzylamino)-cyclohexanol wird in Form seines Hydrochlorids seit Jahrzehnten erfolgreich als sekretlösendes Expektorans eingesetzt [Hagers Handbuch der Pharmazeutischen Praxis (List und Hörhammer Hrsg.), 4. Aufl., 1967 - 1989, Bruchhausen et al., 5. Aufl., 9. Bd, Springer, Berlin 1993 - 1995), (5.) 7, 155 - 159; Pharm. Ztg, 138, (1993) 2754].

Der vorliegenden Erfindung liegt die Aufgabe zugrunde, Ambroxol in eine lokal im Rachenraum wirkende Arzneiform in Gestalt einer Lutschtablette zu bringen. Für die Akzeptanz einer derartigen Lutschtablette durch den Patienten ist neben der Wirksamkeit der sensorische Eindruck ausschlaggebend. Daneben ist ein weiterer wichtiger Punkt die Arzneimittelsicherheit, d.h. es muß sichergestellt sein, daß die vorgesehene Menge an Wirkstoff ohne unerwünschte Neben- bzw. Zersetzungsprodukte, die ggf. nach längerer Lagerung der Zubereitung auftreten können, dem Patienten verabreicht werden kann.

Derartige Zersetzungsprodukte können aus unterschiedlichen Gründen entstehen, beispielsweise durch thermische Labilität der Wirkstoffe oder durch Interaktionen - wie z.B. Zersetzungsreaktionen, die der Wirkstoff mit den pharmazeutischen Hilfsstoffen oder dem Verpackungsmaterial eingeht oder die durch diese Materialien katalysiert werden.

Von dem in der pharmazeutischen Wissenschaft seit vielen Jahren bekannten Wirkstoff Ambroxol waren bislang außer einer Interaktion mit Formaldehyd bzw. Formaldehyd-abspaltenden Verbindungen keine Inkompatibilitäten beschrieben. Neben der Arzneimittelsicherheit und der Wirksamkeit spielt - wie bereits eingangs erwähnt - für die Akzeptanz einer Lutschtablette durch den Patienten unter anderem der gute Geschmack, eine ausreichende Süße und eine angenehme Textur bzw. ein angenehmes Mundgefühl eine wichtige Rolle. Derartige Anforderungen können durch die Wahl entsprechender Hilfsstoffe - wie Aromastoffe oder Süßstoffe - erfüllt werden.

Des weiteren ist zu beachten, daß die Formulierung einer Lutschtablette mit einem zur lokalen Wirkung bestimmten Arzneistoff u.a. einen Matrixbildner erfordert, der gleichmäßig im Munde zergeht und dabei den Wirkstoff freisetzt. Da die Arzneiform längere Zeit dabei mit den Geschmackspapillen in Wechselwirkung tritt, soll der sensorische Eindruck für den Patienten akzeptabel sein. Als Matrixmaterialien werden im Stand der Technik sogenannte Zuckeralkohole eingesetzt.

Unter Zuckeralkoholen versteht man eine Gruppe von Monosacchariden die durch Reduktion der Carbonyl-Funktion erhalten werden. Derartige PolyhydroxyVerbindungen, die keine Zucker sind, gleichwohl aber süß schmecken, finden gewöhnlich als Zuckeraustauschstoffe Verwendung. Man unterscheidet bei diesen im allgemeinen kristallinen wasserlöslichen Polyolen nach der Anzahl der im Molekül enthaltenen Hydroxy-Gruppen Tetrite, Pentite, Hexite usw. Natürlich vorkommende Zuckeralokohle sind z. B. Glycerin, Threit und Erythrit, Adonit (Ribit), Arabit (früher: Lyxit) und Xylit, Dulcit (Galactit), Mannit sowie Sorbit (Glucit bzw. Sorbitol) [Vgl. Römpp, Lexikon Chemie, Georg Thieme Verlag Stuttgart / New York 1998; Belitz-Grosch (3.), S. 701; Karrer, Nr. 139-158, 5401-5410; Kirk-Othmer 1, 569-588; (3.) 1, 754-777].

Daneben sollen derartige Systeme zweckmäßigerweise Hilfsstoffe enthalten, die die technische Handhabbarkeit derartiger Tabletten erleichtern. Beispielsweise ist es nicht erwünscht, daß die Tabletten klebrige Eigenschaften aufweisen, was u.a. dazu führen kann, daß die Gefahr besteht, daß die Tabletten durch Aneinanderkleben oder Kleben an den Werkzeugoberflächen den technischen Herstellungsprozeß beeinträchtigen. Daneben ist es auf Seiten des Patienten unerwünscht, daß sich die Tablette - beispielsweise aufgrund ihrer Klebeeigenschaften - nur schwer aus der Verpackung entfernen läßt. Deshalb werden derartigen Tabletten praktischerweise sogenannte Trenn- oder Schmiermittel zugesetzt. Hierzu werden üblicherweise sogenannte Metallseifen - wie z.B. Calcium- oder Magnesiumstearat oder Calciumarachinat - eingesetzt [U.I. Leinonen, H.U. Jalonen, P.A. Vihervaara, E.S.U.

Laine: Physical and Lubrication Properties of Magnesium Stearate, Journal of Pharmaceutical Sciences 81 (1992) 1194 -1197].

Überraschenderweise stellte sich nun heraus, daß bei der Zubereitung von Ambroxol Hydrochlorid in einer der üblichen aromatisierten Grundlagen auf der Basis von Zuckeralkoholen - insbesondere unter Verwendung von Sorbitol als Matrixmaterial - sowie unter Verwendung von Magnesiumstearat als Trennmittel bei der Stabilitätsprüfung ein bislang nicht in Erscheinung getretenes Nebenprodukt - bislang unaufgeklärter chemischer Struktur - nachweisbar wurde, dessen Entstehung stark von der jeweiligen Lagertemperatur abhängig ist.

Da die Gegenwart eines Neben- oder Zersetzungsproduktes in einem Arzneimittel auf keinen Fall tolerierbar ist, stellt sich somit die Aufgabe, eine Zubereitungsform in Form einer Lutschtablette aufzufinden, die besonders in dieser Hinsicht den Anforderungen der Arzneimittelsicherheit voll Genüge leistet.

Als eine gegebenenfalls gangbare Alternative bietet sich zunächst der Austausch des Matrixmaterials an.

Mit der aus dem Stand der Technik ebenfalls als Matrixmaterial bekannten Saccharose können jedoch nur unter dem Einsatz hoher Preßdrucke Tabletten mit ausreichend hoher Härte gepreßt werden. Weiterhin ist dieser Hilfsstoff kariogen.

Der Einsatz von Sorbitol liefert hingegen Tabletten genügender Härte und ist nicht kariogen. Auf der anderen Seite neigt dieser Zuckeralkohol stark zum Kleben an den Tablettierwerkzeugen, was den Zusatz eines Trennmittels zwingend erforderlich macht.

Überraschenderweise wurde nun gefunden, daß eine ambroxolhaltige Lutschtablette auf der Basis von Zuckeralkoholen insbesondere von Sorbitol als Matrixmaterial unter Verwendung eines Polyethylenglycols und eines pharmazeutisch tolerierbaren Schichtsilikats - insbesondere Talkum - als Schmier- bzw. Trennmittel erhalten werden kann, wobei die Bildung eines Nebenproduktes auch bei längerer Lagerung nicht nachweisbar ist bzw unter der Nachweisgrenze liegt.

Die erfindungsgemäß einsetzbaren Polyethylenglykole besitzen zweckmäßigerweise ein Molekulargewicht in einem Intervall von 3.500 bis 20.000. Bevorzugt wird ein Polyethylenglykol mit einem Molekulargewicht in einem Intervall von 4.000 bis 12.000, wobei ein Polyethylenglykol mit einem Molekulargewicht von ca. 6.000 besonders bevorzugt wird. Ein derartiges Polyethylenglycol ist unter der Bezeichnung Macrogol 6000 aus dem Stand der Technik bekannt.

Unter dem bevorzugt eingesetzten Talkum versteht man im allgemeinen ein - verbreitetes - hydratisiertes Magnesiumsilicat der Zusammensetzung Mg₃[(OH)₂/Si₄O₁₀] oder 3 MgO₄·SiO₂. Es ist aber auch der Einsatz eines anderen aus pharmazeutischer Sicht geeigneten Phyllosilikates möglich.

Der Anteil an Talkum in einer der erfindungsgemäßen Lutschtablette mit einer Gesamtmasse von 1.5 g liegt dabei in einem Bereich von 5 bis 100 mg, bevorzugt 20 bis 80 mg, besonders bevorzugt bei 30 bis 60 mg und ganz besonders bevorzugt bei 60 mg.

Der Anteil an Wirkstoff - im vorliegenden Fall Ambroxol - hängt von der jeweils gewünschten Dosierung ab und ist ebenfalls in einem weiten Bereich variierbar. Zweckmäßigerweise liegt der Anteil an Wirkstoff in einer Lutschtablette in einem Bereich von 1 bis 50 mg, bevorzugt 5 bis 30 mg und besonders bevorgt in einem Intervall von 10 bis 25 mg an Ambroxol in Form seines Hydrochlorids.

Der Anteil der übrigen Hilfs- bzw. Aromastoffe ist unkritisch und läßt sich in weiten Bereichen den jeweiligen Erfordernissen anpassen.

Die nachfolgenden Beispiele erläutern die vorliegende Erfindung, ohne sie auf diese einzuschränken:

### Formulierungsbeispiele:

| Rezeptur A: | |
|---|---|
| Ambroxol HCl : | 10,0 mg |
| Pfefferminz Aroma : | 16,0 mg |
| Saccharin - Na : | 0,5 mg |
| Sorbitol: | 1458,5 mg |
| Magnesiumstearat: | 15,0 mg |
| Gesamtmasse : | 1500,0 mg |

Die Rezepturen A und C sind mit konventioneller Direktverpressungs-Technologie hergestellt.

| Rezeptur B: | |
|---|---|
| Ambroxol HCl : | 10,0 mg |
| Pfefferminz Aroma: | 16,0 mg |
| Saccharin - Na : | 0,5 mg |
| Sorbitol : | 1438,5 mg |
| Macrogol 6000: | 30,0 mg |
| Talkum: | 5,0 mg |
| Gesamtmasse: | 1500,0 mg |

Die Rezptur B wurde hergestellt, indem das Talkum mittels des Verfahrens der Preßkammerbeschichung nur auf die Tablettenoberfläche aufgebracht wurde [Gruber, Gläsel, Liske: Preßkammerbeschichtung, ein Beitrag zur Optimierung der Tablettenherstellung, Pharm Ind. 50 (1988) 839-845].

| Rezeptur C: | |
|---|---|
| Ambroxol HCl : | 20,0 mg |
| Pfefferminz Aroma | 16,0 mg |
| Saccharin - Na : | 0,5 mg |
| Sorbitol: | 1403,5 mg |
| Macrogol 6000 | 30,0 mg |
| Talkum | 30,0 mg |
| Gesamtmasse: | 1500,0 mg |

Die nachfolgende Tabelle gibt die jeweiligen Gehalte an Zersetzungsprodukt der einzelnen Zubereitungen wieder

| Zersetzungsproduktbildung in den Beispielrezepturen in %* | | | | | | |
|---|---|---|---|---|---|---|
| Packmittel: | Polypropylen - Röhren | | | | | |
| Lagerbeding. | 25° C/60 % r.F | | | 30° C/70 % r.F | | |
| Lagerzeit (Mo.) | A | B | C | A | B | C |
| 0 | < 0,2 | < 0,2 | < 0,2 | < 0,2 | < 0,2 | < 0,2 |
| 3 | < 0,2 | < 0,2 | n.d. | 0,27 | < 0,2 | < 0,2 |
| 6 | < 0,2 | < 0,2 | n.d. | 0,34 | < 0,2 | < 0,2 |
| 12 | 0,26 | < 0,2 | n.d. | 0,89 | < 0,2 | < 0,2 |
| 24 | 0,53 | < 0,2 | n.d. | 1,80 | 0,32 | < 0,2 |

| | | | | | | |
|---|---|---|---|---|---|---|
| *): Zersetzungsprodukt in Peakflächen-% bezogen auf den Wirkstoffgehalt; r.F. = relative Feuchte | | | | | | |

Wie der Tabelle eindeutig zu entnehmen ist, liegt der Anteil an Zersetzungsprodukt jeweils unter 0,2 % bzw. unter der Nachweisgrenze.

## Patentansprüche

1. Ambroxolhaltige Lutschtablette auf der Basis von Zuckeralkoholen als Matrixmaterial **gekennzeichnet durch** einen Gehalt eines pharmazeutisch tolerierbaren Schichtsilikats und eines Polyethylenglycols gegebenenfalls neben weiteren pharmazeutischen Hilfs-, Geschmacks- bzw. Aromastoffen.

2. Ambroxolhaltige Lutschtablette nach Anspruch 1, **dadurch gekennzeichnet, daß** das Schichtsilikat Talkum ist.

3. Ambroxolhaltige Lutschtablette nach Anspruch 1 oder 2, **dadurch gekennzeichnet, daß** der Gehalt an Schichtsilikat in einem Bereich von 5 bis 100 mg - bezogen auf eine Gesamtmasse der Tablette von 1,5 g - liegt.

4. Ambroxolhaltige Lutschtablette nach Anspruch 3, **dadurch gekennzeichnet, daß** der Gehalt an Schichtsilikat in einem Bereich von 20 bis 80 mg liegt.

5. Ambroxolhaltige Lutschtablette nach Anspruch 4, **dadurch gekennzeichnet, daß** der Gehalt an Schichtsilikat in einem Bereich von 30 bis 60 mg liegt.

6. Ambroxolhaltige Lutschtablette nach Anspruch 5, **dadurch gekennzeichnet, daß** der Gehalt an Schichtsilikat 60 mg bertägt.

7. Ambroxolhaltige Lutschtablette nach einem der Ansprüche 1 bis 6, **dadurch gekennzeichnet, daß** der Gehalt an Ambroxol Hydrochlorid in einem Bereich von 1 bis 50 mg - bezogen auf eine Gesamtmasse der Tablette von 1,5 g - liegt.

8. Ambroxolhaltige Lutschtablette nach Anspruch 7, **dadurch gekennzeichnet, daß** der Gehallt an Ambroxol Hydrochlorid in einem Bereich von 5 bis 30 mg liegt.

9. Ambroxolhaltige Lutschtablette nach Anspruch 8, **dadurch gekennzeichnet, daß** der Gehalt an Ambroxol Hydrochlorid in einem Bereich 10 bis 25 mg liegt.

10. Ambroxolhaltige Lutschtablette nach einem der Ansprüche 1 bis 9, **dadurch gekennzeichnet, daß** das Polyethylenglykol ein Molekulargewicht in einem Bereich von 3.500 bis 20.000 besitzt.

11. Ambroxolhaltige Lutschtablette nach Anspruch 10, **dadurch gekennzeichnet, daß** das Polyethylenglykol ein Molekulargewicht in einem Bereich von 4.000 bis 12.000 besitzt.

12. Ambroxolhaltige Lutschtablette nach Anspruch 11, **dadurch gekennzeichnet, daß** das Polyethylenglykol ein Molekulargewicht von 6.000 besitzt.

13. Ambroxolhaltige Lutschtablette nach einem der Ansprüche 1 bis 12, **dadurch gekennzeichnet, daß** als Matrixmaterial Sorbitol eingesetzt wird.

14. Ambroxolhaltige Lutschtablette nach einem der Ansprüche 1 bis 13, **dadurch gekennzeichnet, daß** als Süßungsmittel das Natriumsalz des Saccharins eingesetzt wird.

15. Ambroxolhaltige Lutschtablette nach einem der Ansprüche 1 bis 14, **dadurch gekennzeichnet, daß** als Aromastoff Pfefferminzaroma eingesetzt wird.

16. Verfahren zur Herstellung einer ambroxolhaltigen Lutschtablette gemäß einem der Ansprüche 1 bis 15, **dadurch gekennzeichnet, daß** man die einzelnen Bestandteile nach an sich bekannten Verfahrensschritten miteinander vermischt und zu einer Tablette verpresst.

17. Verfahren nach Anspruch 16, **dadurch gekennzeichnet, daß** man die Tablette auf dem Wege der Direktverpressung herstellt.

18. Verfahren nach Anspruch 16, **dadurch gekennzeichnet, daß** man das Schichtsilikat mittels des Presskammerbeschichtungsverfahrens aufbringt.

19. Verwendung der ambroxolhaltigen Lutschtablette gemäß einem der Ansprüche 1 bis 15 zur Herstellung eines Arzneimittels mit sekretlösenden Eigenschaften.

## Claims

1. Tablet for sucking containing ambroxol, based on sugar alcohols as the matrix material, **characterised in that** it contains a pharmaceutically acceptable laminar silicate and a polyethyleneglycol optionally together with other pharmaceutical excipients, taste enhancers or flavourings.

2. Tablet for sucking containing ambroxol according to claim 1, **characterised in that** the laminar silicate is talc.

3. Tablet for sucking containing ambroxol according to claim 1 or 2, **characterised in that** the content of laminar silicate is in the range from 5 to 100 mg, based on a total mass of the tablet of 1.5 g.

4. Tablet for sucking containing ambroxol according to claim 3, **characterised in that** the content of laminar silicate is in the range from 20 to 80 mg.

5. Tablet for sucking containing ambroxol according to claim 4, **characterised in that** the content of laminar silicate is in the range from 30 to 60 mg.

6. Tablet for sucking containing ambroxol according to claim 5, **characterised in that** the content of laminar silicate is 60 mg.

7. Tablet for sucking containing ambroxol according to one of claims 1 to 6, **characterised in that** the content of ambroxol hydrochloride is in the range from 1 to 50 mg, based on a total mass of the tablet of 1.5 g.

8. Tablet for sucking containing ambroxol according to claim 7, **characterised in that** the content of ambroxol hydrochloride is in the range from 5 to 30 mg.

9. Tablet for sucking containing ambroxol according to claim 8, **characterised in that** the content of ambroxol hydrochloride is in the range from 10 to 25 mg.

10. Tablet for sucking containing ambroxol according to one of claims 1 to 9, **characterised in that** the polyethyleneglycol has a molecular weight in the range from 3,500 to 20,000.

11. Tablet for sucking containing ambroxol according to claim 10, **characterised in that** the polyethyleneglycol has a molecular weight in the range from 4,000 to 12,000.

12. Tablet for sucking containing ambroxol according to claim 11, **characterised in that** the polyethyleneglycol has a molecular weight of 6,000.

13. Tablet for sucking containing ambroxol according to one of claims 1 to 12, **characterised in that** sorbitol is used as the matrix material.

14. Tablet for sucking containing ambroxol according to one of claims 1 to 13, **characterised in that** the sodium salt of saccharine is used as the sweetener.

15. Tablet for sucking containing ambroxol according to one of claims 1 to 14, **characterised in that** peppermint flavouring is used as the flavouring.

16. Process for preparing a tablet for sucking containing ambroxol according to one of claims 1 to 15, **characterised in that** the individual components are mixed together using process steps known *per se* and compressed to form a tablet.

17. Process according to claim 16, **characterised in that** the tablet is prepared by direct compression.

18. Process according to claim 16, **characterised in that** the laminar silicate is applied by the compression chamber coating method.

19. Use of the tablet for sucking containing ambroxol according to one of claims 1 to 15 for preparing a pharmaceutical composition with secretion-releasing properties.

## Revendications

1. Comprimé à sucer contenant de l'ambroxol à base d'alcools de sucres comme matériau formant matrice **caractérisé par** une teneur en un silicate feuilleté pharmaceutiquement tolérable et en un polyéthylèneglycol, éventuellement en plus d'autres adjuvants, agents de sapidité et substances aromatisantes pharmaceutiques.

2. Comprimé à sucer contenant de l'ambroxol selon la revendication 1 **caractérisé en ce que** le silicate feuilleté est le talc.

3. Comprimé à sucer contenant de l'ambroxol selon la revendication 1 ou 2 **caractérisé en ce que** la teneur en silicate feuilleté est située dans un domaine de 5 à 100 mg pour une masse totale du comprimé de 1,5 g.

4. Comprimé à sucer contenant de l'ambroxol selon la revendication 3 **caractérisé en ce que** la teneur en silicate feuilleté est située dans un domaine de 20 à 80 mg.

5. Comprimé à sucer contenant de l'ambroxol selon la revendication 4 **caractérisé en ce que** la teneur en silicate feuilleté est située dans un domaine de 30 à 60 mg.

6. Comprimé à sucer contenant de l'ambroxol selon la revendication 5 **caractérisé en ce que** la teneur en silicate feuilleté est de 60 mg.

7. Comprimé à sucer contenant de l'ambroxol selon l'une des revendications 1 à 6 **caractérisé en ce que** la teneur en chlorhydrate d'ambroxol est située dans un domaine de 1 à 50 mg pour une masse totale du comprimé de 1,5 g.

8. Comprimé à sucer contenant de l'ambroxol selon la revendication 7 **caractérisé en ce que** la teneur en chlorhydrate d'ambroxol est située dans un domaine de 5 à 30 mg.

9. Comprimé à sucer contenant de l'ambroxol selon la revendication 8 **caractérisé en ce que** la teneur en chlorhydrate d'ambroxol est située dans un domaine de 10 à 25 mg.

10. Comprimé à sucer contenant de l'ambroxol selon l'une des revendications 1 à 9 **caractérisé en ce que** le polyéthylèneglycol possède une masse moléculaire dans un domaine de 3500 à 20000.

11. Comprimé à sucer contenant de l'ambroxol selon la revendication 10 **caractérisé en ce que** le polyéthylèneglycol possède une masse moléculaire dans un domaine de 4000 à 12000.

12. Comprimé à sucer contenant de l'ambroxol selon la revendication 11 **caractérisé en ce que** le polyéthylèneglycol possède une masse moléculaire dans un domaine de 6000.

13. Comprimé à sucer contenant de l'ambroxol selon l'une des revendications 1 à 12 **caractérisé en ce que** le sorbitol est utilisé comme matériau formant matrice.

14. Comprimé à sucer contenant de l'ambroxol selon l'une des revendications 1 à 13 **caractérisé en ce que** le sel de sodium de la saccharine est utilisé comme édulcorant.

15. Comprimé à sucer contenant de l'ambroxol selon l'une des revendications 1 à 14 **caractérisé en ce que** l'arôme de menthe poivrée est utilisé comme substance aromatisante.

16. Procédé de production d'un comprimé à sucer contenant de l'ambroxol selon l'une des revendications 1 à 15 **caractérisé en ce que** l'on mélange entre eux les différents constituants selon des étapes de procédé connues en soi et on les compresse en un comprimé.

17. Procédé selon la revendication 16 **caractérisé en ce que** l'on produit le comprimé par compression directe.

18. Procédé selon la revendication 16 **caractérisé en ce que** l'on applique le silicate feuilleté au moyen du procédé d'enrobage à enceinte sous pression.

19. Utilisation du comprimé à sucer contenant de l'ambroxol selon l'une des revendications 1 à 15 pour la production d'un médicament à propriétés de fluidification des sécrétions.
